# DEMANDE DE BREVET EUROPEEN

(11) **EP 1 759 689 A2**
(43) Date de publication de la demande: **07.03.2007**
(21) Numéro de dépôt: 06118779.5
(22) Date de dépôt: 11.08.2006
(51) Int. Cl.: A61K 8/42, A61K 8/81, A61Q 19/00

(54) **Emulsion cosmétique comprenant une hydroxyalkylurée et une polyoléfine à partie polaire**

(30) Priorité: 30.08.2005 FR 0552601
(71) Demandeur: L'ORÉAL, 75008 Paris (FR)
(72) Inventeur: Simonnet, Jean-Thierry, 94240, Cachan (FR)
(74) Mandataire: Kromer, Christophe

(57) **Abrégé**

L'invention concerne une composition comprenant une phase aqueuse dispersée dans une phase huileuse, et au moins un composé de formule (I) suivante : 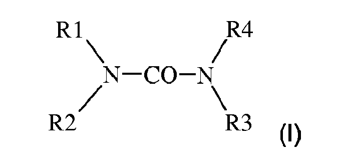 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères,
et au moins un émulsionnant polyoléfine à partie polaire.

Application en cosmétique, notamment soin et au maquillage des matières kératiniques.

## Description

L'invention concerne une émulsion eau-dans-huile comprenant un dérivé d'urée et un émulsionnant polyoléfine à partie polaire, ainsi que l'utilisation de l'émulsion dans les domaines cosmétique ou dermatologique.

La peau humaine est constituée de deux compartiments, à savoir un compartiment superficiel, l'épiderme, et un compartiment profond, le derme. L'épiderme humain naturel est composé principalement de trois types de cellules qui sont les kératinocytes, très majoritaires, les mélanocytes et les cellules de Langerhans.

Chacun de ces types cellulaires contribue par ses fonctions propres au rôle essentiel joué dans l'organisme par la peau. En particulier, les kératinocytes subissent un processus de maturation continu et orienté qui, des kératinocytes se trouvant dans la couche basale de l'épiderme, aboutit la formation de cornéocytes, qui sont des cellules mortes totalement kératinisées constituées de kératinocytes au stade terminal de leur différenciation. Ce processus de différenciation permet d'aboutir la formation des lipides organisés en bicouche dans le stratum corneum. Il permet également de produire les constituants du NMF (Natural Moisturizing Factor) composé de petites molécules hydrosolubles à fort pouvoir hygroscopique jouant un rôle hydratant. Les NMF agissent en outre comme plastifiants, et contribuent ainsi à la souplesse de la peau. Le stratum corneum ainsi constitué joue un rôle capital au niveau de la fonction barrière et de l'hydratation.

On sait que la peau a tendance à se dessécher du fait de facteurs environnementaux (pollution, vent, froid, air conditionné), psychologiques (fatigue, stress) ou hormonaux (ménopause). Or, il est important que la peau soit bien hydratée et ne subisse pas de perte en eau risquant d'entraîner un flétrissement et un dessèchement de la peau.

Aussi, il est courant d'incorporer dans les compositions cosmétiques des humectants, qui sont des substances hygroscopiques qui provoquent une réhydratation de la peau par captation de l'eau atmosphérique et par rétention de l'eau dans la peau. Des exemples de ces hydratants sont les constituants du NMF mentionné précédemment.

Le NMF est principalement constitué d'acides aminés (65%), d'acides organiques (21%) dont l'acide pyrrolidone carboxylique, d'ions (8%), d'urée (4%) et de sucres (2%).

Ainsi, il est connu aussi d'utiliser les acides hydroxylés et leurs sels, et notamment l'acide lactique et le lactate de sodium pour améliorer la souplesse et l'élasticité de la peau (M. Rieger, Cosmetics & Toiletries, 1992, vol. 107, pp.89-90.). L'urée a également été utilisée à cette fin (FR-2 181 659). Il existe par ailleurs dans le commerce des mélanges de constituants du NMF, utilisables dans le domaine cosmétique, tels que les produits vendus sous les dénominations commerciales Hydrasoft par la société VINCIENCE et Hydrosmyl LS4513 par la société COGNIS. Enfin, certains polyholosides hétérogènes sont des précuseurs biologiques de sucres et donc utilisables comme hydratants.

Outre les constituants du NMF, on a aussi utilisé pour améliorer l'hydratation de la peau des polyols tels que le propylène glycol ou la glycérine, et des glycosaminoglycannes (GAG), ou mucopolysaccharides. Ces composés ont une forte capacité de rétention d'eau. Parmi ceux-ci, les GAG sulfatés et l'acide hyaluronique sont synthétisés par les kératinocytes. Outre la solution consistant à apporter ces composés à la peau par voie exogène, il a donc aussi été proposé d'utiliser des composés favorisant leur synthèse (voie endogène).

Il est également connu des demandes DE-A-2703185 et EP-A-1535607 une composition hydratante de soin de la peau et des cheveux comprenant une hydroxyalkyl urée, en particulier la N-(2-hydroxyéthyl)-urée.

Il n'en demeure pas moins qu'il reste le besoin de disposer de compositions ayant une action hydratante améliorée permettant de prévenir et/ou lutter de façon plus efficace contre les signes du dessèchement cutané.

Le but de la présente invention est donc de disposer d'une composition comprenant une hydroxyalkyl urée dont l'action hydratante est améliorée.

Les inventeurs ont découvert que les propriétés hydratantes des dérivés hydroxyalkylurée peuvent être améliorées en formulant ces dérivés dans une émulsion eau-dans-huile contenant comme émulsionnant une polyoléfine à partie polaire.

De façon plus précise, l'invention concerne une composition comprenant une phase aqueuse dispersée dans une phase huileuse, et au moins un composé d'urée de formule (I) suivante : 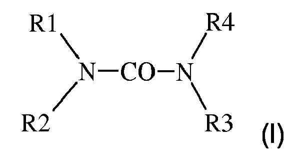 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères,
et au moins un émulsionnant polyoléfine à partie polaire.

L'invention a également pour objet un procédé cosmétique (non thérapeutique) de traitement ou de maquillage des matières kératiniques comprenant l'application sur les matières kératiniques d'une composition telle que décrite précédemment. Le procédé s'applique en particulier pour la peau et les lèvres, notamment pour la peau ou les lèvres sèches.

L'invention a encore pour objet l'utilisation cosmétique d'une composition telle que décrite précédemment pour hydrater les matières kératiniques, en particulier la peau et les lèvres.

Le composé d'urée présent dans la composition selon l'invention est un composé de formule (I) suivante : 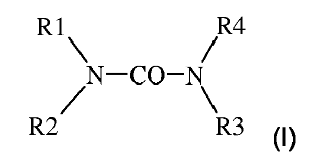 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères.

Pour les composés de formule (I) :
- De préférence R₁ désigne un groupe hydroxyalkyle en C₂-C₆ et R₂, R₃, R₄ désignent, indépendamment l'un de l'autre, un atome d'hydrogène ou un groupe alkyle en C₁-C₄ ;
- Préférentiellement R₁ désigne un groupe hydroxyalkyle en C₂-C₆ comprenant de 1 à 5 groupes hydroxyles, notamment 1 groupe hydroxyle, et R₂, R₃, R₄ désignent un atome d'hydrogène ;
- Plus préférentiellement, R₁ désigne un groupe hydroxyalkyle en C₂-C₄ comprenant 1 groupe hydroxyle et R₂, R₃, R₄ désignent un atome d'hydrogène.

Parmi les groupes alkyle, on peut citer les groupes méthyle, éthyle, n-propyle, iso-propyle, n-butyle, iso-butyle et tert-butyle.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée ; le N-méthyl-N'-(1-hydroxy-2-méthyl-2-propyl)- urée; le N-(1-hydroxy-2-méthyl-2-propyl)- urée ; le N-(1,3-dihydroxy-2-propyl)- urée; le N-(tris-hydroxyméthyl-méthyl)-urée ; le N-éthyl-N'-(2-hydroxyéthyl)- urée; le N,N-bis-(2-hydroxyéthyl)- urée; le N,N'-bis-(2-hydroxyéthyl)- urée; le N,N-bis-(2-hydroxypropyl)- urée; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée ; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée; le N',N'-Bis-(2-hydroxyéthyl)-N',N'-bis-(2-hydroxypropyl)-urée ; et leurs mélanges.

De préférence, le composé de formule (I) est la N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société NATIONAL STARCH sous la dénomination commerciale Hydrovance^{®}.

Le composé de formule (I) peut être présent dans l'émulsion selon l'invention en une teneur allant de 1 % à 50 % en poids, par rapport au poids total de la composition, de préférence allant de 2 % à 25 % en poids, et préférentiellement allant de 2 % à 20 % en poids.

Les polyoléfines à partie polaire utilisables dans l'invention sont connues dans d'autres domaines. Ainsi, elles sont décrites par exemple dans les documents US-A-5,129,972 et US-A-4,919,179, comme stabilisants d'émulsions explosives.
Par ailleurs, ces composés sont connus comme stabilisants de compositions fertilisantes (voir les documents US-A-5,518,517 et US-A-5,858,055) en vue d'obtenir un relargage contrôlé des substances fertilisantes.
Les polyoléfines à partie(s) polaire(s) utilisées dans la composition de l'invention sont constituées d'une partie apolaire polyoléfinique et d'au moins une partie polaire. Elles peuvent présenter une structure de type bloc ou peigne.
La partie apolaire polyoléfinique comprend au moins 40 atomes de carbone et de préférence de 60 à 700 atomes de carbone. Cette partie apolaire peut être choisie parmi les polyoléfines telles que les oligomères, les polymères et/ou les copolymères d'éthylène, d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tetradécène, de 1-pentadécène, de 1-hexadécène, de 1-heptadécène et de 1-octadécène. Ces polyoléfines sont hydrogénées ou non.

Par ailleurs, les polyoléfines à partie polaire utilisées dans l'émulsion de l'invention comportent au moins une partie polaire. Cette partie polaire leur confère des propriétés amphiphiles. Ainsi, ces polyoléfines à partie polaire abaissent la tension interfaciale (eau/huile) d'au moins 10 mN/m quand ils sont présents à une concentration de 0,01% en poids par rapport au poids total de la phase huileuse. Par exemple, la polyoléfine à terminaison succinique commercialisée sous la dénomination Lubrizol 2724 par la société Lubrizol, à une concentration de 0,01% en poids par rapport au poids total de la phase huileuse, abaisse la tension interfaciale de 15 mN/m à l'interface d'une phase aqueuse constituée d'une solution aqueuse à 1% de MgSO₄, et d'une phase huileuse comportant un mélange d'huiles (isohexadécane/polyisobutène hydrogéné/silicone volatile dans un rapport 8/6/4).

La partie polaire des polyoléfines à partie polaire de l'invention peut être anionique, cationique, non ionique, zwitterionique ou amphotère. Elle est par exemple constituée de polyalkylène-glycols ou de polyalkylène-imines, ou encore d'acides ou de diacides carboxyliques, de leurs anhydrides ou de leurs dérivés tels que leurs esters, leurs amides et leurs sels, et leurs mélanges. Les polyoléfines à partie polaire acide carboxylique peuvent être par exemple issues de la réaction entre une polyoléfine et au moins un acide ou anhydride carboxylique éventuellement totalement ou partiellement salifié, choisi dans le groupe comprenant l'acide ou l'anhydride succinique, l'acide maléique, l'anhydride maléique, l'acide fumarique, l'acide itaconique, l'acide citraconique (ou acide méthyl maléique), l'acide mésaconique (ou acide méthyl fumarique), l'acide aconitique, , leurs dérivés esters ou amides, et leurs mélanges.

De préférence, la partie polaire est constituée par l'acide ou l'anhydride succinique, les esters ou amides de l'acide ou de l'anhydride succinique, les sels de métal alcalin ou alcalino-terreux ou sels organiques de l'acide ou de l'anhydride succinique, ou les sels partiels des monoesters ou monoamides de l'acide ou de l'anhydride succinique, ou bien encore par un polyoxyéthylène.

Les polyoléfines à partie polaire polyoxyéthylène peuvent être par exemple choisies parmi les polymères diblocs polyisoprène-polyoxyéthylène, les polymères poly(éthylène-co-propylène)-polyoxyéthylène et leurs mélanges. Ces polymères sont décrits dans la publication de Allgaier, Poppe, Willner, Richter (Macromolecules, 1997, vol. 30, p.1582-1586).

Les polyoléfines à partie polaire acide ou anhydride succinique peuvent être choisies notamment parmi les dérivés polyoléfines d'acide ou d'anhydride succinique décrits dans les brevets US-A-4,234,435, US-A-4,708,753, US-A-5,129,972, US-A-4,931,110, GB-A-2,156,799, US-A-4,877,756 et US-A-4,919,179. La partie polyoléfine peut être constituée par exemple de polyisobutylène, hydrogéné ou non, de poids moléculaire allant de 400 à 5000. Dans le polyisobutylène à terminaison succinique ainsi obtenu, la partie succinique peut être éventuellement modifiée, c'est-à-dire estérifiée, amidifiée ou sous forme de sel. Elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine, de triéthanolamine, de diéthyléthanolamine. Les polyoléfines à terminaison succinique estérifiée ou amidifiée sont des produits de réaction de (a) une polyoléfine à terminaison succinique, et de (b) une amine ou un alcool, pour former une amide ou un ester. Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées. Comme exemple d'alcanolamines, on peut citer la diéthyléthanolamine, la triéthanolamine.

Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatiques, cycloaliphatiques, aromatiques et hétérocycliques. Comme exemples de polyalcool, on peut citer le glycérol.

Les polyoléfines à terminaison succinique modifiée (estérifiée ou amidifiée) et leur procédé de préparation sont décrits en particulier dans le document US-A-4,708,753. On utilise de préférence des polyoléfines à terminaison succinique estérifiée.

Comme polyoléfines à terminaison succinique, on peut citer notamment les poly-isobutylènes à terminaison succinique estérifiée, notamment estérifiée par la diéthanolamine, et leurs sels, notamment les sels de diéthanolamine, tels que les produits commercialisés sous les dénominations Lubrizol® 2724, Lubrizol® 2722 et Lubrizol® 5603 par la société Lubrizol.

Un autre exemple de polyoléfine à partie polaire utilisable dans l'invention est le produit de la réaction de l'anhydride maléique avec le polyisobutylène, tel que les produits commercialisés sous les dénominations Glissopal (Glissopal 2300, 1300 et 1000) (nom INCI : polyisobutene) par la société BASF.

La polyoléfine à partie polaire particulièrement préférée est un produit réactionnel de l'anhydride polyisobuténylsuccinique avec de la diéthyléthanolamine, formant ainsi un sel de diéthyléthanolamine de polybutène succinate de 2-(N, N diéthyl)-amino éthyle. Ce produit est vendu par exemple sous la dénomination Lubrizol® 5603 par la société Lubrizol, et peut être représenté par la formule suivante : 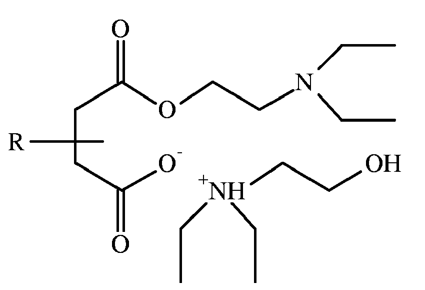 dans laquelle R représente un groupe polyisobutényle, notamment de masse moléculaire en poids de 1000. Ce produit a pour nom INCI : Hydroxyethyldiethonium Polyisobutenyl Triethylaminosuccinate (and) Diethyl ethanolamine.

Une autre polyéoléfine à partie polaire particulièrement préférée est un ester de polyisobutényl succinate de diéthanolaminoéthyle et de triéthanolamnine. Ce produit est vendu par exemple sous la dénomination Chemccinate® 2000 par la société Chemron.

Comme polyoléfine à partie polaire, on peut également utiliser un ester de polyisobutényl succinate de glycéryle, notamment celui vendu sous la dénomination Chemccinate® 1000 AF par la société Chemron.

De préférence, le ou les polyoléfines à partie polaire, telles que définies ci-dessus, sont présentes dans l'émulsion selon l'invention en une teneur allant de 0,01 % à 10 % en poids, de préférence de 0,1 % à 7 % en poids, et mieux encore de 0,2 % à 5 % en poids par rapport au poids total de la composition

La composition selon l'invention contient une phase aqueuse dispersée dans une phase huileuse L'émulsion eau-dans-huile.

Selon un mode préféré de réalisation de l'invention, la phase huileuse de la composition selon l'invention contient au moins une huile. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène (ou squalane) ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl Sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, l'huile de vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- des alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphényl-méthyldiphényl trisiloxanes, les 2-phényléthyltriméthyl-siloxysilicates, et les polyméthylphénylsiloxanes ;
- leurs mélanges.

Selon un mode préféré de réalisation de l'invention, la composition contient au moins une huile choisie parmi les hydrocarbures linéaires ou ramifiés, d'origine minérale ou synthétique, tels que l'huile de Parléam.

La phase huileuse de la composition selon l'invention peut comprendre d'autres corps gras notamment choisis parmi les acides gras comportant de 8 à 30 atomes de carbone, comme l'acide stéarique, l'acide laurique, l'acide palmitique ; les gommes telles que les gommes de silicone (diméthiconol) ; les cires ; les corps gras-pâteux ; et leurs mélanges.

La phase huileuse comprenant tous les corps gras et les adjuvants lipidiques éventuellement présents (par exemple charges, actifs, etc) peut être présente dans la composition selon l'invention en une teneur allant généralement de 5 à 80 %, de préférence de 10 à 60 % en poids et mieux de 10 à 30 % en poids par rapport au poids total de la composition.

La phase aqueuse de la composition de l'invention peut être présente en une teneur allant de 20 à 95 % en poids, par rapport au poids total de la composition, de préférence allant de 50 à 92 % en poids, et mieux allant de 60 à 90 % en poids. Elle contient au moins de l'eau, notamment en une teneur allant de 40 % à 90 % en poids, par rapport au poids total de la composition, et de préférence allant de 50 % à 85 % en poids. Elle peut contenir, outre l'eau, un ou plusieurs solvants miscibles à l'eau ou au moins en partie miscibles à l'eau à la température ambiante.
Par "température ambiante", il faut comprendre une température d'environ 25°C, à pression atmosphérique normale.

Comme solvants miscibles à l'eau, on peut citer notamment les mono-alcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3 butylène glycol et le di-propylène glycol, les cétones en C₃ et C₄ et les aldéhydes en C₂ à C₄

Le ou les solvants miscibles à l'eau peuvent être présents en une quantité allant de 0,1 à 30 % en poids par rapport au poids total de la composition.

La composition de l'invention peut être une émulsion E/H ou encore une émulsion multiple E /H /E. Cette émulsion E/H peut être utilisée telle quelle ou être utilisée pour la préparation d'émulsion multiple E/H/E par incorporation de l'émulsion E/H primaire dans une phase aqueuse externe additionnelle. Ainsi, les émulsionnants polyoléfine à partie polaire décrits ci-dessus peuvent être utilisés aussi comme émulsionnants d'émulsion multiple E/H/E.

La composition de l'invention peut constituer notamment une composition cosmétique, dermatologique ou pharmaceutique et plus particulièrement une composition cosmétique, notamment destinée à une application sur les matières kératiniques, en particulier sur la peau et/ou les muqueuses.

Les compositions de l'invention, peuvent comprendre au moins une matière colorante pouvant notamment être présent à raison de 0,01 % à 40 % en poids, notamment de 0,01 % à 30 % en poids et en particulier de 0,05 % à 25 % en poids, par rapport au poids total du produit.

Les matières colorantes peuvent être choisies parmi les pigments, les colorants hydrosolubles ou liposolubles, les nacres, les paillettes et leurs mélanges.

Par pigments, il faut comprendre des particules blanches ou colorées, minérales ou organiques, insolubles dans la phase hydrophile liquide, destinées à colorer et/ou opacifier la composition. Par nacres, il faut comprendre des particules irisées, notamment produites par certains mollusques dans leur coquille ou bien synthétisées.

On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.
Les colorants hydrosolubles sont par exemple le jus de betterave, le bleu de méthylène.

Les compositions selon l'invention peuvent, de plus, comprendre tous les ingrédients classiquement utilisés dans les domaines concernés et plus spécialement dans le domaine cosmétique et dermatologique. Ces ingrédients sont en particulier choisis parmi les matières colorantes, les vitamines, les antioxydants, les épaississants, les oligo-éléments, les adoucissants, les séquestrants, les parfums, les agents alcalinisants ou acidifiants, les conservateurs, les antioxydants, les filtres UV, les actifs hydrophiles ou lipophiles et leurs mélanges, les charges. Les quantités de ces différents ingrédients sont celles classiquement utilisées dans les domaines concernés et par exemple de 0,01 % à 20 % du poids total de la composition.

De façon connue, la composition selon l'invention peut également contenir des agents actifs habituels dans le domaine cosmétique ou dermatologique. On peut citer en particulier tous les actifs connus pour leur activité sur le vieillissement de la peau comme les agents keratolytiques ou prodesquamants, par exemple les α-hydroxy-acides, les β-hydroxy-acides, les α-ceto-acides, les rétinoïdes et leurs esters, le rétinol, l'acide rétinoïque et ses dérivés On peut citer aussi les vitamines, telles que par exemple les vitamines C, B3 ou PP, B5, E et les dérivés de ces vitamines et notamment leurs esters; la vitamine K et ses dérivés (K1, K2,...); les agents anti-radicaux libres; les filtres solaires; les agents hydratants comme les polyols; la DHEA et ses dérivés; le coenzyme Q10; les agents blanchissants et dépigmentants comme l'acide kojique, les dérivés de para-aminophénols, l'arbutine et leurs dérivés, et leurs mélanges.

Bien entendu, l'homme de l'art veillera à choisir le ou les éventuels composés à ajouter à la composition selon l'invention et leurs quantités, de manière telle que les propriétés avantageuses attachées intrinsèquement à la composition conforme à l'invention ne soient pas ou substantiellement pas, altérées par l'addition envisagée.

La composition selon l'invention peut se présenter notamment sous forme d'une crème, de pommade de lait, de sérum, de lotion.

La composition de l'invention peut être par exemple utilisée pour le soin, le traitement, le maquillage des matières kératiniques (notamment d'être humain), telles que la peau, les lèvres, les cheveux, les cils, les ongles, et en particulier de la peau (notamment du visage, du corps, du cuir chevelu) et des lèvres.

La composition selon l'invention peut être un produit pour le soin de la peau, notamment pour le visage, le cou, le contour de l'oeil, le corps, ou une composition de maquillage, notamment un produit de maquillage des lèvres (rouge à lèvres), un fond de teint, un fard à joues, un fard à paupières, un eye-liner, un produit anticernes, un produit de maquillage du corps.

Avantageusement, la composition est une composition non rincée.

La composition de l'invention peut aussi être utilisée pour le soin et/ou le traitement des cheveux (par exemple masque pour cheveux).

L'invention est illustrée plus en détail dans les exemples décrits ci-après, les teneurs étant exprimées en pourcentage pondéral.

### Exemple 1 :

On a préparé une émulsion eau-dans huile comprenant les ingrédients suivants :

| Phase 1 : | |
|---|---|
| Isohexadécane | 7 % |
| Cyclopentasiloxane | 5 % |
| Isododécane | 2 % |
| Mélange de polyisobutényl succinate de triéthanolamine-diéthanolamine et de palmitate de 2-éthylhexyle (Chemccinate 2000 de Chemron) | 3,5 % |

| Phase 2 : | |
|---|---|
| N-(2-hydroxyéthyl)-urée | 7,5% |
| Sulfate de magnésium | 0,8 % |
| Eau | qsp 100 % |

La phase 2 est introduite sous vive agitation dans la phase 1. On obtient une émulsion eau-dans-huile fluide. L'application de la composition sur le visage permet d'hydrater la peau de manière satisfaisante et sans effet collant.

### Exemple 2 comparatif : hors invention

On a préparé une émulsion similaire à celle de l'exemple 1 en remplaçant le polyisobutényl succinate par un autre émulsionnant, l'isostéarate de polyglycérol-3, ne faisant pas partie de l'invention.

| Phase 1 : | |
|---|---|
| Isohexadécane | 7 % |
| Cyclopentasiloxane | 5 % |
| Isododécane | 2 % |
| Isostéarate de polyglycérol-3 | 3,5 % |

| Phase 2 : | |
|---|---|
| N-(2-hydroxyéthyl)-urée | 7,5 % |
| Sulfate de magnésium | 0,8 % |
| Eau | qsp 100 % |

L'émulsion est préparée de la même manière que celle de l'exemple 1.

L'application de cette émulsion sur la peau confère une performance hydratante inférieur à celle obtenue avec l'émulsion selon l'invention de l'exemple 1.

### Exempte 3 :

On prépare une émulsion eau-dans huile comprenant les ingrédients suivants :

| Phase 1 : | |
|---|---|
| Isohexadécane | 10 % |
| Cyclopentasiloxane | 5 % |
| Isododécane | 2 % |
| Mélange de polyisobutényl succinate de sel de diéthyléthanolamine de polybutène succinate de 2-(N, N diéthyl)-amino éthyle (Lubrizol^{®} 5603 de Lubrizol) | 3,5 % |

| Phase 2 : | |
|---|---|
| N-(2-hydroxyéthyl)-urée | 7,5 % |
| Sulfate de magnésium | 0,8 % |
| Eau | qsp 100 % |

La phase 2 est introduite sous vive agitation dans la phase 1. On obtient une émulsion eau-dans-huile fluide. L'application de la composition sur le visage permet d'hydrater la peau de manière satisfaisante et sans effet collant.

## Revendications

1. Composition comprenant une phase aqueuse dispersée dans une phase huileuse, et au moins un composé de formule (I) suivante : 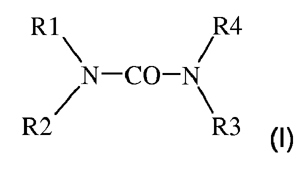 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères,
et au moins un émulsionnant polyoléfine à partie polaire.

2. Composition selon la revendication précédente, **caractérisée par le fait que** pour les composés de formule (I) R₁ désigne un groupe hydroxyalkyle en C₂-C₆ et R₂, R₃, R₄ désignent, indépendamment l'un de l'autre, un atome d'hydrogène ou un groupe alkyle en C₁-C₄.

3. Composition selon l'une quelconque des revendications précédentes, **caractérisée en ce que** pour les composés de formule (I) R₁ désigne un groupe hydroxyalkyle en C₂-C₆ comprenant de 1 à 5 groupes hydroxyles, et R₂, R₃, R₄ désignent un atome d'hydrogène.

4. Composition selon la revendication précédente, **caractérisée en ce que** R₁ désigne un groupe hydroxyalkyle en C₂-C₆ comprenant 1 groupe hydroxyle.

5. Composition selon l'une quelconque des revendications précédentes, **caractérisée par le fait que** pour les composés de formule (I) R₁ désigne un groupe hydroxyalkyle en C2-C4 comprenant 1 groupe hydroxyle et R₂, R₃, R₄ désignent un atome d'hydrogène.

6. Composition selon l'une quelconque des revendications précédentes, **caractérisée par le fait que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée ; le N-méthyl-N'-(1-hydroxy-2-méthyl-2-propyl)- urée ; le N-(1-hydroxy-2-méthyl-2-propyl)- urée ; le N-(1,3-dihydroxy-2-propyl)- urée ; le N-(tris-hydroxyméthyl-méthyl)-urée ; le N-éthyl-N'-(2-hydroxyéthyl)- urée; le N,N-bis-(2-hydroxyéthyl)- urée; le N,N'-bis-(2-hydroxyéthyl)- urée; le N,N-bis-(2-hydroxypropyl)- urée; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée ; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée; le N',N'-Bis-(2-hydroxyéthyl)-N',N'-bis-(2-hydroxypropyl)-urée ; et leurs mélanges.

7. Composition selon l'une quelconque des revendications précédentes, **caractérisée par le fait que** le composé de formule (I) est le N-(2-hydroxyéthyl)-urée.

8. Composition selon l'une quelconque des revendications précédentes, **caractérisée par le fait que** les sels des composés de formule (I) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

9. Composition selon l'une quelconque des revendications précédentes, **caractérisée par le fait que** le composé de formule (I) est présent en une teneur allant de 1 % à 50 % en poids, par rapport au poids total de la composition, de préférence allant de 2 % à 25 % en poids, et préférentiellement allant de 2 % à 20 % en poids.

10. Composition selon l'une quelconque des revendications précédentes, **caractérisée par le fait que** la polyoléfine à partie polaire comporte une partie apolaire polyoléfinique comprenant au moins 40 atomes de carbone, et de préférence de 60 à 700 atomes de carbone.

11. Composition selon la revendication précédente, **caractérisée en ce que** la partie apolaire polyoléfinique est choisie parmi les oligomères, les polymères et/ou les copolymères d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tetradécène, de 1-pentadécène, de 1-hexadécène, de 1-heptadécène et de 1-octadécène.

12. Composition l'une quelconque des revendications précédentes, **caractérisée en ce que** la partie polaire de la polyoléfine est anionique, cationique, non ionique, zwitterionique ou amphotère.

13. Composition selon l'une quelconque des revendications précédentes, **caractérisée en ce que** la partie polaire de la polyoléfine est constituée de polyalkylène-glycols, de polyalkylène-imines, d'acides ou de diacides carboxyliques, de leurs anhydrides ou dérivés tels que esters, amides et sels, et leurs mélanges.

14. Composition selon la revendication précédente, **caractérisée en ce que** la partie polaire de la polyoléfine est choisie dans le groupe comprenant le polyoxyéthylène, l'acide ou l'anhydride succinique, les esters ou amides de l'acide ou de l'anhydride succinique, les sels de métal alcalin ou alcalino-terreux ou sels organiques de l'acide ou de l'anhydride succinique, ou les sels partiels des monoesters ou monoamides de l'acide ou de l'anhydride succinique.

15. Composition selon l'une quelconque des revendications précédentes, **caractérisée en ce que** la polyoléfine à partie polaire est un polyisobutylène à terminaison succinique éventuellement modifiée.

16. Composition selon l'une quelconque des revendications précédentes, **caractérisée en ce que** la polyoléfine à partie polaire est le un polyisobutylène à terminaison succinique estérifiée.

17. Composition selon l'une quelconque des revendications précédentes, **caractérisée par le fait que** la polyoléfine à partie polaire est présente en une teneur allant de 0,01 % à 10 % en poids, de préférence de 0,1 % à 7 % en poids, et mieux encore de 0,2 % à 5 % en poids par rapport au poids total de la composition

18. Composition selon l'une quelconque des revendications précédentes, **caractérisée par le fait qu'**elle comprend une huile.

19. Composition selon l'une quelconque des revendications précédentes, **caractérisée par le fait que** la phase huileuse est présente en une teneur allant de 5 à 80 % en poids, par rapport au poids total de la composition, de préférence de 10 à 60 % en poids et mieux de 10 à 30 % en poids.

20. Composition selon l'une quelconque des revendications précédentes, **caractérisée par le fait que** la phase aqueuse est présente en une teneur allant de 20 à 95 % en poids, par rapport au poids total de la composition, de préférence allant de 50 à 92 % en poids, et mieux allant de 60 à 90 % en poids.

21. Composition selon l'une quelconque des revendications précédentes, **caractérisée par le fait qu'**elle est une émulsion eau-dans-huile ou une émulsion multiple E/H/E.

22. Composition selon l'une quelconque des revendications précédentes, **caractérisée par le fait qu'**elle comprend un ingrédient cosmétique choisi parmi les matières colorantes, les vitamines, les antioxydants, les épaississants, les oligo-éléments, les adoucissants, les séquestrants, les parfums, les agents alcalinisants ou acidifiants, les conservateurs, les antioxydants, les filtres UV, les actifs hydrophiles ou lipophiles et leurs mélanges, les charges.

23. Composition selon l'une quelconque des revendications précédentes, **caractérisée par le fait qu'**elle constitue une composition cosmétique ou dermatologique.

24. Procédé cosmétique non thérapeutique de traitement ou de maquillage des matières kératiniques comprenant l'application sur les matières kératiniques d'une composition selon l'une quelconque des revendications précédentes.

25. Utilisation cosmétique d'une composition selon l'une quelconque des revendications 1 à 23 pour hydrater les matières kératiniques.
